# FASCICULE DE BREVET EUROPEEN

(11) **EP 1 173 179 B1**
(45) Date de publication et mention de la délivrance du brevet: **19.03.2003**
(21) Numéro de dépôt: 00922735.6
(22) Date de dépôt: 25.04.2000
(51) Int. Cl.: A61K 31/451, A61P 25/18, A61P 25/24

(54) **UTILISATION DU SAREDUTANT ET DE SES SELS PHARMACEUTIQUEMENT ACCEPTABLES DANS LE TRAITEMENT OU LA PREVENTION DES TROUBLES DEPRESSIFS MAJEURS**
VERWENDUNG DES SAREDUTANT UND SEINER PHARMAZEUTISCH AKZEPTABLEN SALZE ZUR BEHANDLUNG VON SCHWERE DEPRESSION
USE OF SAREDUTANT AND THE PHARMACEUTICALLY ACCEPTABLE SALTS THEREOF TO PRODUCE MEDICAMENTS USED TO TREAT OR PREVENT MAJOR DEPRESSIVE DISORDERS

(30) Priorité: 27.04.1999 FR 9905338
(43) Date de publication de la demande: 23.01.2002
(73) Titulaire: SANOFI-SYNTHELABO, 75013 Paris (FR)
(72) Inventeur: EMONDS-ALT, Xavier, F-34980 Combaillaux (FR); SOUBRIE, Philippe, F-34270 Valflaunes (FR); STEINBERG, Régis, F-34730 Prades le Lez (FR)
(74) Mandataire: Thouret-Lemaitre, Elisabeth
(86) Numéro de dépôt international: FR0001084
(87) Numéro de publication internationale: WO00064423

(56) Documents cités:
- WO-A-94/16697
- HAGAN R.M. ET AL: "Vineyard peptide conference bears fruit." TRENDS IN PHARMACOLOGICAL SCIENCES, (1993) 14/9 (315-318). , XP000960587
- TEIXEIRA R M ET AL: "Effects of central administration of tachykinin receptor agonists and antagonists on plus-maze behavior in mice." EUROPEAN JOURNAL OF PHARMACOLOGY, (1996 SEP 5) 311 (1) 7-14. , XP000964735
- KHAWAJA A.M. ET AL: "Tachykinins: Receptor to effector." INTERNATIONAL JOURNAL OF BIOCHEMISTRY AND CELL BIOLOGY, (1996) 28/7 (721-738). , XP000964743
- CULMAN J ET AL: "Effect of tachykinin receptor inhibition in the brain on cardiovascular and behavioral responses to stress." JOURNAL OF PHARMACOLOGY AND EXPERIMENTAL THERAPEUTICS, (1997 JAN) 280 (1) 238-46. , XP000964757
- IKEDA K ET AL: "RP67580, a neurokinin1 receptor antagonist, decreased restraint stress-induced defecation in rat." NEUROSCIENCE LETTERS, (1995 SEP 29) 198 (2) 103-6. , XP000964614

## Description

La présente invention a pour objet une nouvelle utilisation du sarédutant.

Sarédutant est la Dénomination Commune Internationale (D.C.I.) du (S)-(-)-N-méthyl-N-[4-(4-acétylamino-4-phénylpipéridin-1-yl)-2-(3,4-dichlorophényl)butyl]benzamide de formule :

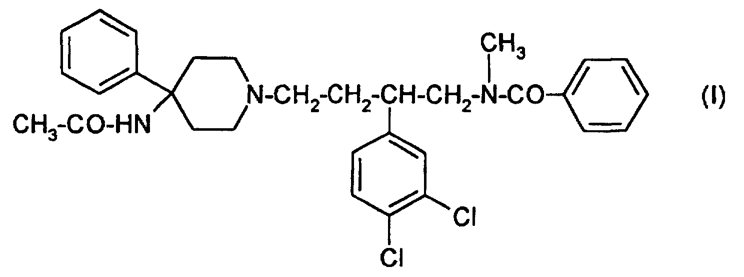

Ce composé et ses sels pharmaceutiquement acceptables sont décrits dans le brevet EP 0 474 561 B1 et dans le brevet US 5 236 921.

Ces composés sont décrits comme des antagonistes de la neurokinine A et peuvent être utiles dans toute pathologie neurokinine A dépendante et plus particulièrement dans les inflammations neurogéniques des voies respiratoires. Ces composés ont également été décrits comme des antagonistes non peptidiques, puissants et sélectifs des récepteurs NK₂ de la neurokinine A (Life Sciences, 1992, 50 (15), PL101-PL106).

On a maintenant trouvé que le sarédutant et ses sels pharmaceutiquement acceptables sont utiles dans le traitement ou la prévention des troubles dépressifs majeurs.

La présente invention a pour objet l'utilisation du sarédutant et de ses sels pharmaceutiquement acceptables pour la préparation de médicaments utiles dans le traitement ou la prévention des troubles dépressifs majeurs.

Le sarédutant et ses sels pharmaceutiquement acceptables sont préparés selon le procédé décrit dans le brevet EP 0 474 561 B1 ou celui décrit dans le brevet EP 0 698 601 B1.

Les sels du composé de formule (I) sont les sels avec des acides minéraux ou organiques pharmaceutiquement acceptables classiques tels que le chlorhydrate, le bromhydrate, le sulfate, l'hydrogénosulfate, le dihydrogénophosphate, le méthanesulfonate, le méthylsulfate, l'acétate, l'oxalate, le maléate, le fumarate, le succinate, le naphtalène-2-sulfonate, le glyconate, le gluconate, le citrate, l'iséthionate, le benzènesulfonate, le paratoluènesulfonate.

Pour leur utilisation en tant que médicaments, le composé de formule (I) et ses sels pharmaceutiquement acceptables sont généralement administrés en unités de dosage. Lesdites unités de dosage sont de préférence formulées dans des compositions pharmaceutiques dans lesquelles le principe actif est mélangé avec un excipient pharmaceutique.

Dans les compositions pharmaceutiques de la présente invention pour l'administration orale, sublinguale, inhalée, sous-cutanée, intramusculaire, intraveineuse, transdermique, locale ou rectale, le principe actif seul ou en association avec un autre principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topiques, les implants, les formes d'administration sous-cutanée, transdermique, intramusculaire, intraveineuse, intranasale et les formes d'administration rectale.

Le dosage journalier du composé de formule (I) est de 0,05 à 5 mg/kg, avantageusement de 1 à 2,5 mg/kg, préférentiellement de 2 à 2,5 mg/kg, à administrer en une ou plusieurs fois. Le composé de formule (I) et ses sels sont généralement formulés en unité de dosage contenant de 2,5 à 500 mg, avantageusement de 50 à 250 mg, préférentiellement de 100 à 250 mg, de principe actif par unité de dosage, à administrer une fois, deux fois ou plusieurs fois en même temps, selon la nécessité. Bien que ces dosages soient des exemples de situation moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Lorsque l'on prépare une composition solide sous forme de comprimés, on ajoute au principe actif, micronisé ou non, un véhicule pharmaceutique qui peut être composé de diluants comme par exemple le lactose, la cellulose microcristalline, l'amidon et des adjuvants de formulation comme des liants (polyvinylpyrrolidone, hydroxypropylméthylcellulose, etc...), des agents d'écoulement comme la silice, des lubrifiants comme le stéarate de magnésium, l'acide stéarique, le tribéhénate de glycérol, le stéarylfumarate de sodium.

Des agents mouillants ou tensioactifs tels que le laurylsulfate de sodium peuvent être ajoutés à la formulation.

Les comprimés peuvent être réalisés par différentes techniques, compression directe, granulation sèche, granulation humide, fusion à chaud.

Les comprimés peuvent être nus ou dragéifiés (par du saccharose par exemple) ou enrobés avec divers polymères ou autres matières appropriés.

Les comprimés peuvent avoir une libération flash, retardée ou prolongée en réalisant des matrices polymériques ou en utilisant des polymères spécifiques au niveau du pelliculage.

On obtient une préparation en gélule par simple mélange du principe actif avec des véhicules pharmaceutiques secs (simple mélange ou granulation sèche, humide, fusion à chaud), liquides ou semi-solides.

Les gélules peuvent être molles ou dures, pelliculées ou non de manière à avoir une activité flash, prolongée ou retardée (par exemple par une forme entérique).

Une préparation sous forme de sirop ou d'élixir peut contenir le principe actif conjointement avec un édulcorant, acalorique de préférence, du méthylparaben et du propylparaben comme antiseptique, ainsi qu'un agent donnant du goût et un colorant approprié.

Les poudres ou les granules dispersibles dans l'eau peuvent contenir le principe actif en mélange avec des agents de dispersion, des agents mouillants ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale ou intranasale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant comme par exemple un alcool tel que l'éthanol ou un glycol tel que le polyéthylèneglycol ou le propylèneglycol, et un tensioactif hydrophile tel que le Tween® 80. Pour préparer une solution huileuse injectable par voie intramusculaire, on peut solubiliser le principe actif par un triglycéride ou un ester de glycérol.

Pour l'administration locale on peut utiliser des crèmes, des pommades, des gels, des collyres.

Pour l'administration transdermique, on peut utiliser des patches sous forme multilaminée ou à réservoir dans lequel le principe actif peuvent être en solution alcoolique.

Pour une administration par inhalation on utilise un aérosol contenant par exemple du trioléate de sorbitane ou de l'acide oléique ainsi que du trichlorofluorométhane, du dichlorofluorométhane, du dichlorotétrafluoroéthane ou tout autre gaz propulseur biologiquement compatible ; on peut également utiliser un système contenant le principe actif seul ou associé à un excipient, sous forme de poudre.

Le principe actif peut être également présenté sous forme de complexe avec une cyclodextrine, par exemple, α, β, γ-cyclodextrine, 2-hydroxypropyl-β-cyclodextrine, méthyl-β-cyclodextrine.

Le principe actif peut être formulé également sous forme de microcapsules ou microsphères, éventuellement avec un ou plusieurs supports ou additifs.

Parmi les formes à libération prolongée utiles dans le cas de traitements chroniques, on peut utiliser les implants. Ceux-ci peuvent être préparés sous forme de suspension huileuse ou sous forme de suspension de microsphères dans un milieu isotonique.

Selon la présente invention, les formes orales d'administration sont préférées.

L'effet du sarédutant sur les troubles dépressifs majeurs est étudié sur des patients agés de 18 à 65 ans. Les patients reçoivent du sarédutant par voie orale (300 mg/jour) pendant une période de six semaines environ.

L'amélioration des syndromes dépressifs est mesurée par une diminution significative des scores sur l'échelle d'évaluation de la dépression d'Hamilton (HAM-D) ainsi que par le recueil des impressions du clinicien et des impressions globales du patient. L'échelle d'évaluation de la dépression d'Hamilton (Hamilton Depression Rating Scale) est définie par Hamilton M. dans J. Neurol. Neurosurg. Psychiat., 1960, 23, 56-62.

Dans les Exemples suivants, le sarédutant est utilisé sous forme de monosuccinate.

### EXEMPLE 1 : Gélule à 25 mg de sarédutant.

| | | |
|---|---|---|
| Sarédutant (exprimé en base) | | 25,0 mg |
| lactose monohydrate (200 mesh) | QSP | 170 mg |
| croscarmellose sodium | | 3,4 mg |
| stéarate de magnésium | | 1,7 mg |
| eau purifié* | QS | |
| pour une gélule blanc opaque de taille 3, remplie à | | 170 mg |

| | | |
|---|---|---|
| * évaporée au séchage après la granulation humide. | | |

### EXEMPLE 2 : Gélule à 100 mg de sarédutant.

| | | |
|---|---|---|
| Sarédutant (exprimé en base) | | 100,0 mg |
| lactose monohydrate (200 mesh) | QSP | 170 mg |
| croscarmellose sodium | | 3,4 mg |
| stéarate de magnésium | | 1,7 mg |
| eau purifié* | QS | |
| pour une gélule blanc opaque de taille 3, remplie à | | 170 mg |

| | | |
|---|---|---|
| * évaporée au séchage après la granulation humide. | | |

## Revendications

1. Utilisation du saredutant et de ses sels pharmaceutiquement acceptables pour la préparation de médicaments utiles dans le traitement ou la prévention des troubles dépressifs majeurs.

## Claims

1. Use of saredutant and of its pharmaceutically acceptable salts for the preparation of medicinal products that are useful in the treatment or prevention of major depressive disorders.

## Patentansprüche

1. Verwendung von Saredutant und seiner pharmazeutisch annehmbaren Salze für die Herstellung von Arzneimitteln zur Behandlung oder der Vorbeugung von Depressionsstörungen.
